# EUROPEAN PATENT SPECIFICATION

(11) **EP 2 598 124 B1**
(45) Date of publication and mention of the grant of the patent: **04.04.2018**
(21) Application number: 11748604.3
(22) Date of filing: 22.07.2011
(51) Int. Cl.: A61K 9/20, A61K 9/24, A61K 31/404, A61K 31/4045, A61P 25/00

(54) **THREE-PHASE CONTROLLED-RELEASE TABLET COMPRISING MELATONIN AND PROCESS OF PREPARATION**
DREIPHASIGE RETARD-TABLETTE MIT MELATONIN UND HERSTELLUNGSVERFAHREN
COMPRIMÉ À LIBÉRATION CONTRÔLÉE À TROIS PHASES COMPRENANT DE LA MÉLATONINE ET PROCÉDÉ DE PRÉPARATION

(30) Priority: 26.07.2010 IT MI20101374
(43) Date of publication of application: 05.06.2013
(73) Proprietor: Ambros Pharma S.r.l., 20122 Milano (IT)
(72) Inventor: STANKOV, Bojidar Mihaylov, I-20146 Milan (IT)
(74) Representative: Cattaneo, Elisabetta
(86) International application number: PCT/EP2011/062634
(87) International publication number: WO 2012/013595

(56) References cited:
- US-A1- 2004 043 065
- US-A1- 2008 171 085
- US-A1- 2008 254 121
- US-B1- 6 723 342

## Description

### FIELD OF THE INVENTION

The present invention relates to a three-phase controlled-release tablet comprising melatonin and a process for the preparation thereof.

### PRIOR ART

Melatonin (N-acetyl-5-methoxytryptamine) is an indole compound of wide natural occurrence. It is produced in almost all living organisms, from algae to humans. Although in lower organisms melatonin might be part of the natural defences against oxidative stress, it has been established that in mammals and in humans, melatonin is a secondary zeitgeber ("time-giver"), which, in concert with light (primary zeitgeber), is able to sincronize the endogenous biological clock with the phase of the prevailing photoperiod.

In this way melatonin governs the diurnal (circadian) cycles of the organism, displaying a strong synchronizing effect of the rhythm of sleep and wakefulness. To be able to fulfill its function, melatonin must be present in the organism for prolonged periods of time (5-7 hours) only at night and above a threshold physiological concentration. Due to its prominent activity on the circadian clock, melatonin has been used for treating syndromes associated with desynchronization of the body's rhythms of sleep and wakefulness, for example jet-lag, delayed sleep phase syndrome (DSPS), in the blind, in the elderly and in shift worker insomnia.

The natural rhythm of melatonin and its concentration in the peripheral blood decrease dramatically with age, just as when taking certain drugs, for example beta-mimetics, beta-blockers, benzodiazepines, or through taking caffeine, as well as in situations of acute and chronic stress in general psycho-physiological insomnias. If bioavailable at night, melatonin is in fact able to act favourably on the microstructure (CAP and CAP rate) of non-REM sleep, objective parameters for the restorative quality of sleep.

In the past, melatonin was thought to possess mild hypnotic activity. This activity of melatonin was corroborated by data demonstrating that even at a dose of 5 mg, given as it is, it not only does not influence the initial stage of sleep but in some cases has a negative influence on the subsequent stages, for example, on the time of wakefulness after the onset of sleep (wake after sleep onset, WASO) or on the number of awakenings after sleep onset in (NA).

Moreover, in normal conditions melatonin is a powerful regulator of body temperature, and since lowering of body temperature is extremely important for the induction and quality of sleep, this makes a further contribution to its effects on sleep.

Nocturnal release of melatonin in normal conditions occurs with levels above 50-100 pg/ml and, during physiological sleep, lasts about 6-7 hours. The onset of release in normal conditions is always very rapid and the maximum physiological levels are reached within 15-30 minutes from the start of the nocturnal increase in melatonin. In studies of its effects on sleep, this phenomenon determined selection of the various dosages (0.1, 1, 10, 100 mg), selection also being dictated by the short half-life of melatonin (about 40 minutes in humans).

Accordingly, the use of low dosages (0.1, 1 mg), which would correspond to the "physiological" levels of melatonin in the peripheral blood, have proved barely effective in insomnia. However, the administration of high dosages is to be avoided, for reasons associated with the potential prolonged presence of melatonin in the peripheral blood even after waking, when the physiological levels normally are, and should be, very low.

There have been attempts to overcome all of the foregoing by administering melatonin in various sustained-release forms (oral, transcutaneous, transmucosal), which do not, however, reproduce the physiological "pattern" of melatonin in the body. Moreover, the "pulse-release" forms developed to date are very expensive, being produced as preparations constituted of several tablets arranged in the same capsule. Furthermore, they are not suitable for the preparation of dietetic products or products intended for long-term administration, since they use excipients that are not permitted in foodstuffs and food supplements, and may potentially have undesirable effects in the long term.

Document IT1318524 describes tablets which, because of their flexibility of administration, make it possible to vary the release parameters with the possibility of obtaining a precise two-phase release pattern: first, a rapid release of a predetermined percentage, within about 10 minutes, followed by gradual release of the remaining melatonin contained in the tablet.

According to the teaching of document IT1318524 these tablets containing melatonin comprise a slow-release internal core, with predetermined hardness and content of active ingredient, and a quick-release external coating ("cortex"), containing a further predetermined dose of melatonin. The melatonin content described may vary between 0.1 and 100 mg and, in one of the preferred embodiments, between 2 and 3 mg in the internal core and between 0.5 and 1.5 mg in the external "cortex". These tablets have found useful application in the dietetic and/or food sector, for replacement of melatonin that is reduced or lacking in certain circumstances already mentioned above, regulation of the sleep/wake cycle and in control of sleep.

The controlled-release tablets described in IT1318524 are prepared by separately mixing the ingredients for the core, obtaining an internal core of the desired hardness, and the ingredients of the external cortex, obtaining a film (coating) solution and then, applying said coating solution on the internal core under pressure. The release of the total melatonin contents from the tablets obtained amounts to about 30% in the first 10 minutes and more than 80% within four hours.

However, once produced, these tablets should display very precise release characteristics both immediately after production, and following periods of storage at room temperature for the next 24 months. These tablets must in fact display overall release of the melatonin of about 25-40% within 10 minutes and more than 90% within six hours.

These release specifications must be maintained not only when the tablets have just been produced, but after 24 months and, preferably, after 36 months of storage at room temperature.

The tablets produced as in IT1318524 met the specifications when just produced, and 12 months later, but they did not completely satisfy the quality requirements after 24 months, and above all at 36 months of storage. In particular, the tablets produced in example 1 of document IT1318524 showed release of about 18.3% within 10 minutes and of about 72% within 6 hours, after 36 months of storage.

Therefore one of the aims of the present invention is to supply tablets that meet the specifications of maintaining substantially unaltered the release even after 24 months or at 36 months of storage.

### SUMMARY OF THE INVENTION

In an aspect the present invention provides for a three-phase melatonin-releasing tablet according to claim 1.

In another aspect, the present invention relates to a process for the preparation of a tablet according to claim 1 having an internal core and external coating, both comprising melatonin at equal or different dosages, said internal core and external coating being separated by an internal coating which does not comprise melatonin, said process comprising the following steps:
1) preparing the mixture of the core by the following substeps:
   i) wet granulating a mixture of the following ingredients: melatonin, dicalcium phosphate dihydrate, mannitol, a first portion of silicon dioxide with a granulating solution of polyvinylpyrrolidone previously dissolved in a solution of water and ethyl alcohol;
   ii) drying the granulate in a oven at about 60°C until a humidity content of the granulate below 1% is reached;
   iii) calibrating the dried granulate on a mesh screen of about 0.7 mm;
   iv) adding to the calibrated granulate the remaining part of silicon dioxide, hydroxypropylmethylcellulose and lactose (or dicalcium phosphate dihydrate or another suitable pharmaceutically acceptable excipient) previously sieved with sieve with mesh of about 0.7 mm;
   v) mixing the product of substep iv)
   vi) adding magnesium stearate previously sieved with a sieve with a mesh of about 0.7 mm to the mixture of step v) and mixing the product again;
2) preparing the mixture of the external coating by the following substeps:
   a) mixing melatonin, hydroxypropylmethylcellulose, microcrystalline cellulose, a mixture of acetic esters of mono- and di-glycerides of fatty acids of glycerol and titanium dioxide;
   b) suspending the mixture of step a) in depurated water and ethyl alcohol;
   c) preparing, separately from the suspension of step b), a solution of ethanol soluble edible resin dissolved in ethyl alcohol;
3) compressing the product of step 1 until a hardness value in the range from 3 to 6 kN is reached;
4) coating the product of step 3) with the solution of an ethanol soluble edible resin of step 2)-c), and
5) coating the product of step 4) with the suspension of step 2)-b.

In the present invention when reference is made to amounts of an ingredient, when the expression "a first portion" of a particular ingredient is used, this means the use of at least 5 wt.% of the total weight of said ingredient, the expression "the remaining part" of said ingredient referring to the use of at most 95 wt.% of the total weight of said ingredient.

Surprisingly, the tablet of the invention showed a three-phase release profile of the total active ingredient: of about 25-40% within 10 minutes (first phase), an equilibrium phase between 10 and 30 minutes (second phase) and a phase of gradual release of the remaining melatonin (third phase), to reach a cumulative release greater than 90% of the active ingredient within six hours thus simulating the release of endogenous melatonin even after 24 months or at 36 months of storage.

In certain embodiments the slow releasing core a) of the tablet comprises melatonin and at least an excipient, the internal coating b) is melatonin-free and comprises an alcohol-soluble edible resin, and the external coating c) comprises melatonin and an excipient.

For purposes of the present application, the term "edible" is intended to mean food grade materials which are approved by regulatory authorities for use in pharmaceutical or food applications.

The terminology alcohol-soluble edible resin as used herein refers to edible resins which are soluble in ethanol and are used in the pharmaceutical technology to make a film covering a substrate containing a pharmaceutically active ingredient.

Shellac is a typical example of an alcohol-soluble edible resin suitable for the uses of the present invention.

For the purposes of the present application suitable pharmaceutically acceptable excipients comprise sugars, such as lactose; microcrystalline cellulose and cellulose derivatives, pharmaceutically acceptable polymers, pharmaceutically acceptable inorganic salts and lubricants.

Suitable cellulose derivatives may be chosen among hydroxypropylmethyl cellulose, methyl cellulose, hydroxypropyl cellulose, hydroxylethyl cellulose, carboxymethyl cellulose, ethylhydroxyethyl cellulose, cellulose acetate butyrate, cellulose acetate phtalate and their mixtures.

Suitable pharmaceutically acceptable polymers include
- natural polymers, such as natural proteins, in particular gelatine, albumin, natural gums and their mixtures,
- synthetic polymers, preferably chosen among:
   acrylates, in particular polymethacrylates, poly(hydroxy ethyl methacrylate), poly(methyl methacrylate), poly(hydroxy ethyl methacrylate) -co-methyl methacrylate);
- polyamides, in particular polyacrylamide,
- polyanhydrides, in particular poly(bis carboxy phenoxy) methane;
- block (or stabilizing) polymers of the PEO-PPO type, in particular polyoxamers; polyvinylchloride, polyvinyl alcohol, silicones, polyurethanes, synthetic gums, polyethylene oxides, polyesters, and polymers belonging to the lactam family, polyalcohols, amides, oxides and salts. In certain embodiments a polymer belonging to the lactam family is used, in particular pyrrolidone and its derivatives, such as polyvinyl pyrrolidone, polyvinyl polypyrrolidone and their mixtures.

Suitable inorganic salts include calcium or dicalcium phosphate, and their mixtures.

Suitable lubricants includes magnesium stearate, triacylglycerols and their mixtures.

In certain embodiments, the excipients are in an amount of 85 to 99% by weight of total weight of the tablet.

In certain embodiments the alcohol-soluble edible resin of the internal coating is shellac.

The tablet is provided with a core and two coatings as claimed in claim 1, wherein said internal core comprises the following ingredients:

| Ingredients of the core | Amount by weight (%) |
|---|---|
| Melatonin | 0.01 to 5%, preferably 0.5 to 3% |
| Lactose | 10 to 60%, preferably 30 to 50% |
| Hydroxypropylmethylcellulose | 5 to 30 %, preferably 10 to 20% |
| Dicalcium phosphate dihydrate | 5 to 30%, preferably 10 to 20% |
| Mannitol | 5 to 30%, preferably 10 to 20% |
| Polyvinylpyrrolidone | 0.1 to 10%, preferably 1 and 5% |
| Magnesium stearate | 0.01 to 5%, preferably 0.5 to 3% |
| Silicon dioxide | 0.01 to 3%, preferably 0.1 to 2% |

The internal coating comprising

| | |
|---|---|
| Shellac (or other alcohol-soluble edible resin) | Amount by weight (%) |
| | 0.01-3%, preferably 0.1-2% |

The external coating comprising

| Ingredients of the coating | Amount by weight (%) |
|---|---|
| Hydroxypropylmethylcellulose | 0.01 to 10%, preferably 0.5 to 5% |
| Microcrystalline cellulose | 0.01 to 3%, preferably 0.1 to 2% |
| Titanium dioxide | 0.01 to 3%, preferably 0.1 to 2% |
| Mixture of acetic esters of mono- and di-glycerides of fatty acids of glycerol | 0.01 to 2%, preferably 0.1 to 1% |
| Melatonin | 0.01 to 3%, preferably 0.5 to 2% |

### DETAILED DESCRIPTION OF THE DRAWING

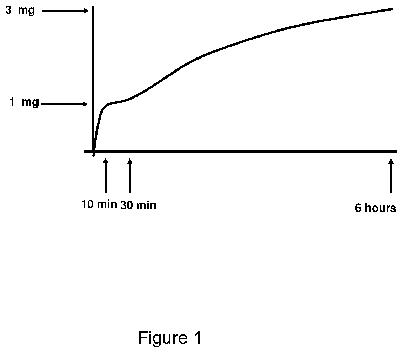

Fig. 1 is a representation of the three-phase release profile of the tablet according to the invention, containing a total of 3 mg melatonin.

The tablet of the invention with core and two coatings displays a three-phase release profile of the active ingredient as illustrated in Fig. 1: overall of about 25-40% within 10 minutes (first phase), an equilibrium phase between 10 and 30 minutes (second phase) and a phase of gradual release of the remaining melatonin (third phase), to reach a cumulative release greater than 90% of the active ingredient within six hours, thus simulating the endogenous melatonin release.

### DETAILED DESCRIPTION OF THE INVENTION

The invention relates to a a tablet according to claim 1.

In another aspect, the present invention relates to a process for the preparation of a tablet according to claim 1 having an internal core and external coating, both comprising melatonin at equal or different dosages, said internal core and external coating being separated by an internal coating which does not comprise melatonin, said process comprising the steps as claimed in claim 6.

Preferably in step 1), the mixture is mixed for about 10 minutes before being treated with the granulating solution.

After the treatment with the granulating solution, the mixture is advantageously kneaded for 25 minutes before being granulated with a granulator.

In step 1)iv), instead of lactose, if this component must not be used, it can be replaced with the same amount of dicalcium phosphate dihydrate or another suitable pharmaceutically acceptable excipient.

The mixing in step 1)v) is preferably carried out for about 15 minutes.

The melatonin content can be between 0.1 and 100 mg both in the internal core and in the external "coating". It can be preferably between 2 and 3 mg in the internal core and between 0.5 and 3 mg in the external coating.

The amounts of all the remaining ingredients of the tablet, added as in the order in the process of the invention, were not limiting. Typically, they are added in the amounts stated in the examples, although amounts falling in a range obtained by increasing or decreasing said amounts by 20% were nevertheless preferred.

The tablets of the invention therefore comply with the release profile of the desired specifications in the physiological environment for the predetermined periods of time. Surprisingly, the release profile of the tablet of the invention was three-phase. In particular, the tablets of the invention thus obtained displayed three-phase release characteristics of the active ingredient: overall of about 25-40% in the first 10 minutes (first phase), an equilibrium phase between 10 and 30 minutes (second phase) and a phase of gradual release to reach cumulative release greater than 90% within six hours (third phase) thus simulating the release of endogenous melatonin even after 12, 24 months or 36 months of storage.

In certain embodiments a first releasing phase occurs in the tablets within 10 minutes with a release profile of 25-40% of total melatonin, a second releasing or an equilibrium phase occurs from about 10 to about 30 minutes and a third releasing phase occurs by six hours after 24 months of storage with a release profile of more than 90% of total melatonin.

In certain embodiments a first releasing phase occurs in the tablets within 10 minutes with a release profile of 25-40% of total melatonin, a second releasing or equilibrium phase occurs from about 10 to about 30 minutes and a third releasing phase occurs by six hours after 36 months of storage with a release profile of more than 90% of total melatonin.

In an addition aspect the tablet according to the invention for use as a medicament is provided.

In certain embodiments the tablet is applicable in the treatment of sleep disorders. In certain embodiments the tablet of the invention is suitable for the treatment of defects in TST, NA and WASO in psychophysiological insomnia.

In certain embodiments the tablet is useful in the treatment of the sleep stability.

In additional embodiments the tablets of the invention are applicable in the treatment of drug addicted patients who show sleep disorders.

According to additional embodiments the tablets are useful is the treatment of patients who do not respond to the administration of a conventional melatonin-based tablet.

The following examples are provided merely for illustrating the present invention and are not to be intended as limiting the scope of protection as results from the appended claims.

### Experimental section

### Example 1

### 1) preparation of the core

The following ingredients were used in the stated amounts:

| Ingredient | Percentage by weight of the whole tablet (%) |
|---|---|
| Melatonin | 1.09 |
| Lactose | 40.76 |
| Hydroxypropylmethylcellulose | 16.85 |
| Dicalcium phosphate dihydrate | 16.30 |
| Mannitol | 16.85 |
| Polyvinylpyrrolidone | 2.45 |
| Vegetable magnesium stearate | 0.90 |
| Silicon dioxide | 0.46 |

A mixture was prepared, sieved on a sieve with mesh of 1 mm, of: melatonin, dicalcium phosphate dihydrate, mannitol and a first portion of silicon dioxide. The polyvinylpyrrolidone was dissolved in a solution of water and ethyl alcohol. The ingredients of the mixture were then loaded in a mixer and mixed for 10 minutes. The mixture was thus soaked with the granulating solution of polyvinylpyrrolidone, previously dissolved in a solution of water and ethyl alcohol, and kneaded for 25 minutes. The mixture was then granulated with a granulator attachment with 2 mm screen. The granulate was dried in a stove at 60°C until moisture content of the granulate below 1% was reached and the dried granulate was calibrated on a mesh screen of 0.7 mm;
The remaining part of silicon dioxide, hydroxypropylcellulose and lactose previously sieved with sieve with mesh of 0.7 mm were added to the calibrated granulate. The product was mixed for 15 minutes. Magnesium stearate previously sieved with sieve with mesh of 0.7 mm was added to the mixture and the product was mixed again for 5 minutes.

### 2) Preparation of the coating

The following ingredients were used in the stated amounts:

| Ingredients of the internal coating | Percentage (%) by weight of the whole tablet |
|---|---|
| Shellac | 0.48 |

| Ingredients of the external coating | Percentage (%) by weight of the whole tablet |
|---|---|
| Hydroxypropylmethylcellulose | 1.83 |
| Microcrystalline cellulose | 0.50 |
| Titanium dioxide | 0.66 |
| Mixture of acetic esters of mono- and di-glycerides of fatty acids of glycerol (E472a) | 0.33 |
| Melatonin | 0.54 |

A mixture was prepared comprising melatonin, hydroxypropylmethylcellulose, microcrystalline cellulose, E472a (corresponding to a mixture of acetic esters of mono- and di-glycerides of fatty acids of glycerol) and titanium dioxide. A suspension of said mixture in purified water and ethyl alcohol was then prepared. Separately from the suspension, a solution of shellac dissolved in ethyl alcohol was prepared.

The core prepared in 1) was compressed to hardness in the range from 3 to 6 kN. The core was then treated with the solution of shellac, and the varnished product was coated with the suspension.

Tablets having the following parameters were obtained:
Weight: about 184 mg: 176 in the core and 8 in the coating, comprising a total of 3 mg of melatonin.

The tablet thus obtained was analysed by dissolution test and the corresponding release profile is presented in Fig. 1.

As can be seen from Fig. 1, the tablet showed a three-phase release profile of the active ingredient: overall of about 25-40% released within 10 minutes (first phase), followed by an equilibrium phase between 10 and 30 minutes (second phase) and a phase of gradual release of the remaining melatonin (third phase), to reach a cumulative release greater than 90% of the active ingredient within six hours.

### Example 2

### Evaluation of storage of the tablet according to example 1 and comparison with the tablet produced as in example 1 of IT1318524

In detail, the tablets of the prior art were prepared using the following ingredients for the core and for the coating according to the procedure described in example 1 of IT1318524.

**Ingredients of the core of the prior art**

| Ingredients of the granulate | amount mg |
|---|---|
| melatonin | 2 |
| mannitol | 31 |
| dicalcium phosphate | 30 |
| polyvinylpyrrolidone | 4.5 |
| aerosil 200 | 0.5 |

| Core | amount mg |
|---|---|
| granulate (as above) | 68 |
| hydroxypropylmethylcellulose | 31 |
| lactose | 75 |
| aerosil 200 | 0.35 |
| magnesium stearate | 1.65 |

**Ingredients of the coating**

| Ingredient | amount by weight (%) |
|---|---|
| melatonin | 2.7 |
| hydroxypropyl methyl cellulose | 8.8 |
| lactose | 6.4 |
| titanium dioxide | 0.8 |
| ethyl alcohol | 17.3 |
| purified water | 64 |

The coating solution was applied on the core under pressure.

The tablets obtained had the following parameters:
Weight: about 180 mg (as the sum of the weights of the two layers)

The tablets obtained according to the prior art described in IT1318524 were compared with the tablets obtained as in example 1 of the present invention.

In detail, the tablets were assessed for release both immediately after preparation and after storage in their original primary blisters at room temperature for 12, 24 months and 36 months, respectively.

To comply with the storage specifications the tablets had to display overall release characteristics of the active ingredient of about 25-40% in the first 10 minutes and cumulative release greater than 90% within six hours, to simulate the release of endogenous melatonin.

The results shown in Table 1 for the tablet according to the invention and in Table 2 for the tablet described in IT1318524 were obtained.

**Table 1: Tablet of the invention**

| Time | Specification Cumulative release (%) | Immediately after production Cumulative release (%) | After 12 months of storage Cumulative release (%) | After 24 months of storage Cumulative release (%) | After 36 months of storage Release Cumulative release*^{a)}* (%) |
|---|---|---|---|---|---|
| 10 min | 25-40 | 27.2 | 28.4 | 33.4 | 33.1 |
| 1st hour | 40-60 | 52.3 | 46.9 | 56.0 | 50.5 |
| 2nd hour | 60-75 | 64.9 | 64.3 | 69.7 | 62.0 |
| 4th hour | 75-90 | 79.5 | 78.4 | 87.0 | 86.5 |
| 6th hour | > 90 | 95.0 | 100.1 | 109.4 | 99.0 |

**Table 2: Tablet of the prior art**

| Time | Specification Cumulative release (%) | Immediately after production Cumulative release (%) | After 12 months of storage Cumulative release (%) | After 24 months of storage Cumulative release (%) | After 36 months of storage Cumulative release (%) |
|---|---|---|---|---|---|
| 10 min | 25-40 | 32.3 | 30.1 | 27.2 | 18.3 |
| 1st hour | 40-60 | 53.1 | 46.0 | 42.1 | 32.1 |
| 2nd hour | 60-75 | 72.0 | 68.5 | 59.0 | 51.0 |
| 4th hour | 75-90 | 82.0 | 78.0 | 74.0 | 62.5 |
| 6th hour | > 90 | 98.0 | 93.5 | 88.0 | 72.0 |

| | | | | | |
|---|---|---|---|---|---|
| a) The mean values in Table 1 were significantly different from the respective mean values in the corresponding time intervals in Table 2: (p<0.05 for the values at 36 months of storage). The mean values were obtained from 6 independent determinations (sextuplicates) in the same analysis. | | | | | |

As can be seen from the comparison, the tablet of the invention displayed a release profile of melatonin within the required values at all time points tested, and in particular at 36 months whereas the tablet of the prior art did not reach a cumulative release of 90%.

Since the tablet as produced had a three-phase release profile of melatonin as presented in Fig. 1, the profile of the tablet of the invention was evaluated at 12 months, 24 and 36 months over the preferred dose range. A comprehensive summary of the results are given in Tables 3 and 4.

**Table 3: Tablet of the invention containing 2 mg of melatonin in the core and 1 mg of melatonin in the external coating**

| Time | Immediately after production Cumulative release (%) | After 12 months of storage Cumulative release (%) | After 24 months of storage Cumulative release (%) | After 36 months of storage Cumulative release (%) |
|---|---|---|---|---|
| 10 min | 29.7 | 31.0 | 27.9 | 26.8 |
| 30 min | 32.8 | 33.7 | 32.1 | 31.3 |
| 1st hour | 54.6 | 52.9 | 55.5 | 53.9 |
| 2nd hour | 70.2 | 71.1 | 66.2 | 66.0 |
| 4th hour | 81.6 | 82.0 | 77.9 | 80.3 |
| 6th hour | 101.4 | 103.1 | 98.1 | 98.0 |

**Table 4: Tablet of the invention containing 3 mg of melatonin in the core and 2 mg of melatonin in the external coating**

| Time | Immediately after production Cumulative release (%) | After 12 months of storage Cumulative release (%) | After 24 months of storage Cumulative release (%) | After 36 months of storage Cumulative release (%) |
|---|---|---|---|---|
| 10 min | 31.2 | 31.0 | 30,5 | 31.3 |
| 30 min | 35.0 | 33.5 | 33.3 | 33.8 |
| 1st hour | 46.3 | 45.5 | 45.0 | 45.2 |
| 2nd hour | 60.1 | 59,3 | 65.6 | 64,5 |
| 4th hour | 78.0 | 79.6 | 79.1 | 80.7 |
| 6th hour | 98.0 | 96.3 | 96.0 | 100.6 |

As can be seen from Tables 3 and 4 the tablet of the invention maintained the three-phase profile at 12, 24 and 36 months: immediate melatonin release of about 25-40% within 10 minutes (first phase), equilibrium phase between 10 and 30 minutes (second phase),, followed by a gradual release of the remaining melatonin to reach a total cumulative release greater than 90% within 6 hours (third phase).

Moreover, the tablets prepared according to the invention were assessed for the effects on sleep in humans. On administration, all the patients treated, suffering from psychophysiological insomnia, showed a total sleep time (TST), sleep latency (SL), wake after sleep onset (WASO), number of awakenings (NA) with parameters that were significantly improved following the treatment, similar to the results obtained with the tablets of IT1318524, but post-hoc analysis demonstrated that the results obtained with the tablets of the invention were significantly better: TST increased, but most importantly, NA and WASO decreased by 30% and 25%, (p<0,05) respectively, compared to the results obtained with the tablets of IT1318524. The sleep efficiency (SE) reached unforeseen values superior to 95%. This was a significant unexpected result demonstrating a notable improvement in the sleep parameters, in particular the sleep stability (NA, WASO) and the sleep quality (SE).

The same results were advantageously found if the tablets of the present invention with shelf life of 36 months were administered to drug-addicted patients who had sleep disturbances in the course of a drug-withdrawal treatment or to patients with psychophysiological insomnia who had not responded to therapy with melatonin in the treatment of sleep disorders (negative biased subjects). TSL, SL, NA and WASO were significantly improved in more than 95% of the subjects.

## Claims

1. A three-phase melatonin-releasing tablet comprising
- an internal core comprising:
| Ingredients of the core | Amount by weight (%) |
|---|---|
| Melatonin | 0.01 to 5% |
| Lactose | 10 to 60% |
| Hydroxypropylmethylcellulose | 5 to 30 % |
| Dicalcium phosphate dihydrate | 5 to 30% |
| Mannitol | 5 to 30% |
| Polyvinylpyrrolidone | 0.1 to 10% |
| Magnesium stearate | 0.01 to 5% |
| Silicon dioxide | 0.01 to 3% |
- an internal coating comprising
| | |
|---|---|
| Shellac | Amount by weight (%) |
| | 0.01-3% |
- an external coating comprising
| Ingredients of the coating | Amount by weight (%) |
|---|---|
| Hydroxypropylmethylcellulose | 0.01 to 10% |
| Microcrystalline cellulose | 0.01 to 3% |
| Titanium dioxide | 0.01 to 3% |
| Mixture of acetic esters of mono- and di-glycerides of fatty acids of glycerol | 0.01 to 2% |
| Melatonin | 0.01 to 3% |

2. A three-phase melatonin-releasing tablet according to claim 1 for use as a medicament.

3. A three-phase melatonin-releasing tablet according to claim 1 for use in the treatment of sleep disorders.

4. A three-phase melatonin-releasing tablet according to claim 1 for use as a nutritional supplement or food.

5. A three-phase melatonin-releasing tablet according to claim 1 for use in the treatment of TST (total sleep time), sleep latency (SL), sleep efficiency (SE), NA (number of awakenings) and WASO (wake after sleep onset) in psychophysiological insomnia.

6. A process for the preparation of a tablet according to claim 1 having an internal core and external coating, both comprising melatonin at equal or different dosages, said internal core and external coating being separated by an internal coating which does not comprise melatonin, said process comprising the following steps:
1) preparing a mixture of the core through the following substeps:
i) wet granulating a mixture of the following ingredients: melatonin, dicalcium phosphate dihydrate, mannitol, a first portion of silicon dioxide with a granulating solution of polyvinylpyrrolidone previously dissolved in a solution of water and ethylic alcohol;
ii) drying the granulate in oven at 60°C until a humidity of the granulate below 1% is reached;
iii) calibrating the dried granulate on a mesh screen of about 0.7 mm;
iv) adding to the calibrated granulate the remaining part of silicon dioxide, hydroxypropylmethylcellulose and lactose (or dicalcium phosphate dihydrate or another suitable pharmaceutically acceptable excipient) previously sieved with sieve with mesh of about 0.7mm;
v) mixing the product of the substep iv);
vi) adding magnesium stearate previously sieved with a sieve with a mesh of about 0.7mm to the mixture of step v) and mixing again the product;
2) preparing the suspension of the coating through the following substeps:
a) mixing melatonin, hydroxypropylmethylcellulose, microcrystalline cellulose, a mixture of acetic esters of mono- and di-glycerides of fatty acids of glycerol and titanium dioxide;
b) suspending the mixture of step a) in depurated water and ethylic alcohol;
c) separately of the mixture of step b), preparing a solution of an ethanol soluble edible resin dissolved in ethylic alcohol;
3) compressing the product of step 1) until an hardness value in the range of 3 to 6 kN is reached;
4) coating the product of step 3) with a solution of an ethanol soluble edible resin of step 2) c); and
5) coating the product of step 4) with the suspension of step 2)-b).

7. Process according to claim 6, wherein the melatonin is in amounts of between 2 to 3 mg in the internal core and in amounts of between 0.5 to 3 mg in the external coating.

8. Process according to anyone of claims 6-7 wherein the ethanol soluble edible resin is shellac.

## Patentansprüche

1. Dreiphasige Melatonin-freisetzende Tablette, aufweisend:
- einen inneren Kern, aufweisend:
| **Inhaltsstoffe des Kerns** | **Gewichtsmenge (%)** |
|---|---|
| Melatonin | 0,01 bis 5 % |
| Laktose | 10 bis 60 % |
| Hydroxypropylmethylcellulose | 5 bis 30 % |
| Dikalziumphosphat-Dihydrat | 5 bis 30 % |
| Mannitol | 5 bis 30 % |
| Polyvinyl-Pyrrolidon | 0,1 bis 10 % |
| Magnesiumstearat | 0,01 bis 5 % |
| Siliziumdioxid | 0,01 bis 3 % |
- eine Innenbeschichtung, aufweisend:
| | |
|---|---|
| Schellack | **Gewichtsmenge (%)** |
| | 0,01 bis 3 % |
- eine Außenbeschichtung, aufweisend:
| **Inhaltsstoffe der Beschichtung** | **Gewichtsmenge (%)** |
|---|---|
| Hydroxypropylmethylcellulose | 0,01 bis 10 % |
| mikrokristalline Zellulose | 0,01 bis 3 % |
| Titandioxid | 0,01 bis 3 % |
| Mischung von Essigsäureestern von Mono- und Diglyceriden von Fettsäuren von Glyzerin | 0,01 bis 2 % |
| Melatonin | 0,01 bis 3 % |

2. Dreiphasige Melatonin-freisetzende Tablette gemäß Anspruch 1 zur Verwendung als Medikament.

3. Dreiphasige Melatonin-freisetzende Tablette gemäß Anspruch 1 zur Verwendung bei der Behandlung von Schlafstörungen.

4. Dreiphasige Melatonin freisetzende Tablette gemäß Anspruch 1 zur Verwendung als Nahrungsergänzungsmittel oder Lebensmittel.

5. Dreiphasige Melatonin-freisetzende Tablette gemäß Anspruch 1 zur Verwendung bei der Behandlung von TST (Gesamt-Schlafzeit), Schlaf-Latenz (ST), Schlafeffizienz (SE), NA (Schlafunterbrechungen) und WASO (Aufwachen nach Einsetzen des Schlafs) bei psychophysiologischen Schlafstörungen.

6. Verfahren zur Herstellung einer Tablette gemäß Anspruch 1 mit einem inneren Kern und einer Außenbeschichtung, wobei beide Melatonin mit gleicher oder unterschiedlicher Dosierung aufweisen, und der innere Kern und die Außenbeschichtung durch eine innere Beschichtung getrennt sind, die kein Melatonin aufweist und das Verfahren die folgenden Schritte aufweist:
1) Zubereiten einer Mischung für den Kern durch die folgenden Unterschritte:
i) Nassgranulation einer Mischung der folgenden Inhaltsstoffe:
Melatonin, Dikalziumphosphat-Dihydrat, Mannitol, einen ersten Teil von Siliziumdioxid mit einer Granulationslösung von Polyvinyl-Pyrrolidon, die zuvor in einer Lösung von Wasser und Ethylalkohol gelöst wurde;
ii) Trocknen des Granulats im Ofen bei 60°C bis eine Feuchte des Granulats von unter 1% erreicht ist;
iii) Kalibrieren des getrockneten Granulats durch ein Maschensieb von etwa 0,7 mm;
iv) Zugabe des restlichen Teils Siliziumdioxid, Hydroxypropylmethylcellulose und Laktose (oder Dikalziumphosphat-Dihydrat oder eines anderen pharmazeutisch geeigneten Hilfsstoffs), welche zuvor mit einer Maschenweite von etwa 0,7 mm gesiebt wurde, zu dem kalibrierten Granulat;
v) Mischen des Produkts aus Unterschritt iv);
vi) Zugabe von Magnesiumstearat, das zuvor mit einer Maschenweite von etwa 0,7 mm gesiebt wurde, zur Mischung aus Schritt v) und nochmaliges Mischen des Produkts;
2) Zubereiten der Suspension für die Beschichtung durch folgende Unterschritte:
a) Mischen von Melatonin, Hydroxypropylmethylcellulose, mikrokristalliner Zellulose, einer Mischung von Essigsäureestern von Mono- und Diglyceriden von Fettsäuren von Glyzerin und Titandioxid;
b) Lösen der Mischung aus Schritt a) in gereinigtem Wasser und Ethylalkohol;
c) getrennt von der Mischung aus Schritt b), Zubereiten einer Lösung eines Ethanol-löslichen Speiseharzes, das in Ethylalkohol gelöst ist;
3) Pressen des Produkts aus Schritt 1) bis ein Härtewert im Bereich von 3 bis 6 kN erreicht ist;
4) Beschichtung des Produkts aus Schritt 3) mit der Lösung eines Ethanol-löslichen Speiseharzes aus Schritt 2)c); und
5) Beschichtung des Produkts aus Schritt 4) mit der Lösung aus Schritt 2)b).

7. Verfahren gemäß Anspruch 6, wobei das Melatonin im inneren Kern in einer Menge von 2 bis 3 mg und in der Außenbeschichtung in einer Menge von 0,5 bis 3 mg vorliegt.

8. Verfahren gemäß einem der Ansprüche 6 bis 7, wobei das Ethanol-lösliche Speiseharz Schellack ist.

## Revendications

1. Comprimé de libération de mélatonine à trois phases comprenant
- un noyau interne comprenant :
| Composants du noyau | Quantité en poids (%) |
|---|---|
| Mélatonine | 0,01 à 5 % |
| Lactose | 10 à 60 % |
| Hydroxypropylméthylcellulose | 5 à 30 % |
| Phosphate dicalcique dihydraté | 5 à 30 % |
| Mannitol | 5 à 30 % |
| Polyvinylpyrrolidone | 0,1 à 10 % |
| Stéarate de magnésium | 0,01 à 5 % |
| Dioxyde de silicium | 0,01 à 3 % |
- un enrobage interne comprenant
| Gomme laque | Quantité en poids (%) |
|---|---|
| | 0,01 à 3 % |
- un enrobage externe comprenant
| Composants de l'enrobage | Quantité en poids (%) |
|---|---|
| Hydroxypropylméthylcellulose | 0,01 à 10 % |
| Cellulose microcristalline | 0,01 à 3 % |
| Dioxyde de titane | 0,01 à 3 % |
| Mélanges d'esters acétiques de mono- et di-glycérides d'acides gras de glycérol | 0,01 à 2 % |
| Mélatonine | 0,01 à 3 % |

2. Comprimé de libération de mélatonine à trois phases selon la revendication 1 pour une utilisation en tant que médicament.

3. Comprimé de libération de mélatonine à trois phases selon la revendication 1 pour une utilisation dans le traitement des troubles du sommeil.

4. Comprimé de libération de mélatonine à trois phases selon la revendication 1 pour une utilisation en tant que complément nutritionnel ou aliment.

5. Comprimé de libération de mélatonine à trois phases selon la revendication 1 pour une utilisation dans le traitement du TST (temps de sommeil total), de la LE (latence à l'endormissement), de l'ES (efficacité du sommeil), du NR (nombre de réveils) et du WASO (éveil après endormissement) dans l'insomnie psychophysiologique.

6. Procédé pour la préparation d'un comprimé selon la revendication 1 comportant un noyau interne et un enrobage externe, les deux comprenant de la mélatonine à des dosages égaux ou différents, lesdits noyau interne et enrobage externe étant séparés par un enrobage interne qui ne comprend pas de mélatonine, ledit procédé comprenant les étapes suivantes :
1) la préparation d'un mélange du noyau par les sous-étapes suivantes :
i) la granulation humide d'un mélange des composants suivants : la mélatonine, le phosphate dicalcique dihydraté, le mannitol, une première partie du dioxyde de silicium avec une solution de granulation de polyvinylpyrrolidone dissoute auparavant dans une solution d'eau et d'alcool éthylique ;
ii) le séchage du granulat dans un four à 60 °C jusqu'à ce qu'une humidité du granulat inférieure à 1 % soit atteinte ;
iii) la calibration du granulat séché sur un tamis d'une taille de maille d'environ 0,7 mm ;
iv) l'addition au granulat calibré de la partie restante du dioxyde de silicium, de l'hydroxypropylméthylcellulose et du lactose (ou de phosphate dicalcique dihydraté ou d'un autre excipient pharmaceutiquement acceptable approprié) tamisé auparavant avec un tamis d'une taille de maille d'environ 0,7 mm ;
v) le mélange du produit de la sous étape iv) ;
vi) l'addition du stéarate de magnésium tamisé auparavant avec un tamis d'une taille de maille d'environ 0,7 mm au mélange de l'étape v) et le mélange à nouveau du produit ;
2) la préparation de la suspension de l'enrobage par les sous-étapes suivantes :
a) le mélange de la mélatonine, de l'hydroxypropylméthylcellulose, de la cellulose microcristalline, d'un mélange d'esters acétiques de mono- et di-glycérides d'acides gras de glycérol et du dioxyde de titane ;
b) la mise en suspension du mélange de l'étape a) dans de l'eau et de l'alcool éthylique dépurés ;
c) séparément du mélange de l'étape b), la préparation d'une solution d'une résine comestible soluble dans l'éthanol dissoute dans de l'alcool éthylique ;
3) la compression du produit de l'étape 1) jusqu'à ce qu'une valeur de dureté dans la plage de 3 à 6 kN soit atteinte ;
4) l'enrobage du produit de l'étape 3) avec une solution d'une résine comestible soluble dans l'éthanol de l'étape 2) c) ; et
5) le revêtement du produit de l'étape 4) avec la suspension de l'étape 2) b).

7. Procédé selon la revendication 6, dans lequel la mélatonine est dans des quantités situées entre 2 et 3 mg dans le noyau interne et dans des quantités situées entre 0,5 et 3 mg dans l'enrobage externe.

8. Procédé selon l'une quelconque des revendications 6 ou 7 dans lequel la résine comestible soluble dans l'éthanol est la gomme laque.
